# EUROPEAN PATENT APPLICATION

(11) **EP 2 784 083 A1**
(43) Date of publication of application: **01.10.2014**
(21) Application number: 13161634.4
(22) Date of filing: 28.03.2013
(51) Int. Cl.: C07K 14/51

(54) **Bone Morphogenetic Protein (BMP) variants with highly reduced antagonist sensitivity and enhanced specific biological activity**

(71) Applicant: Charité - Universitätsmedizin Berlin, 10117 Berlin (DE)
(72) Inventor: Seemann, Petra, 10965 Berlin (DE); Zimmer, Julia, 64390 Erzhausen (DE); Duda, Georg, 12209 Berlin (DE); Hecht, Jochen, 14129 Berlin (DE); Degenkolbe, Elisa, 10317 Berlin (DE)
(74) Representative: Gruber, Daniel

(57) **Abstract**

Instant invention refers to peptides having the biological activity of a BMP molecule comprising or consisting of an amino acid sequence having an amino acid identity of at least 50% of SEQ ID No. 1, wherein a first amino acid substitution is at position N59 of SEQ ID No. 1 and a second amino acid substitution is at position N102 of SEQ ID No. 1, nucleic acids encoding for the said peptides, vectors comprising the said nucleic acid, transgenic host cells or organisms, a process for the manufacturing of the peptide of the invention, pharmaceutical compositions, research tools or diagnostic tools comprising the molecules, vectors or cells of the invention, the use of the molecules of the invention for the manufacturing of a research tool or diagnostic tool, or for the prevention, treatment or diagnosis of a BMP-related disease or condition.

SEQUENCE LISTING

<110> Charité - Universitätsmedizin Berlin

<120> Bone Morphogenetic Protein (BMP) variants with highly reduced
antagonist sensitivity and enhanced specific biological activity

<160> 7

<170> BiSSAP 1.0

<210> 1
<211> 114
<212> PRT
<213> Homo sapiens

<220>
<221> SOURCE
<222> 1..114
<223> /mol_type="protein"
      /note="Mature human BMP2 "
      /organism="Homo sapiens"

<400> 1
Gln Ala Lys His Lys Gln Arg Lys Arg Leu Lys Ser Ser Cys Lys Arg
1               5                   10                  15
His Pro Leu Tyr Val Asp Phe Ser Asp Val Gly Trp Asn Asp Trp Ile
            20                  25                  30
Val Ala Pro Pro Gly Tyr His Ala Phe Tyr Cys His Gly Glu Cys Pro
        35                  40                  45
Phe Pro Leu Ala Asp His Leu Asn Ser Thr Asn His Ala Ile Val Gln
    50                  55                  60
Thr Leu Val Asn Ser Val Asn Ser Lys Ile Pro Lys Ala Cys Cys Val
65                  70                  75                  80
Pro Thr Glu Leu Ser Ala Ile Ser Met Leu Tyr Leu Asp Glu Asn Glu
                85                  90                  95
Lys Val Val Leu Lys Asn Tyr Gln Asp Met Val Val Glu Gly Cys Gly
            100                 105                 110
Cys Arg

<210> 2
<211> 41
<212> DNA
<213> artificial sequences

<220>
<221> source
<222> 1..41
<223> /mol_type="DNA"
      /note="Primer"
      /organism="artificial sequences"

<400> 2
ggctgatcat ctgaactcca ctgttcatgc cattgttcag a                          41

<210> 3
<211> 41
<212> DNA
<213> artificial sequences

<220>

<221> source
<222> 1..41
<223> /mol_type="DNA"
      /note="Primer"
      /organism="artificial sequences"

<400> 3
tctgaacaat ggcatgaaca gtggagttca gatgatcagc c                          41


<210> 4
<211> 54
<212> DNA
<213> artificial sequences

<220>
<221> source
<222> 1..54
<223> /mol_type="DNA"
      /note="Primer"
      /organism="artificial sequences"

<400> 4
ccttgacgag aatgaaaagg ttgtattaaa gtggtatcag gacatggttg tgga            54


<210> 5
<211> 54
<212> DNA
<213> artificial sequences

<220>
<221> source
<222> 1..54
<223> /mol_type="DNA"
      /note="Primer"
      /organism="artificial sequences"

<400> 5
tccacaacca tgtcctgata ccactttaat acaacctttt cattctcgtc aagg            54


<210> 6
<211> 1191
<212> DNA
<213> Homo sapiens

<220>
<221> source
<222> 1..1191
<223> /mol_type="DNA"
      /note="coding sequence of pre-pro-protein of human BMP2"
      /organism="Homo sapiens"

<400> 6
atggtggccg ggacccgctg tcttctagcg ttgctgcttc cccaggtcct cctgggcggc       60

gcggctggcc tcgttccgga gctgggccgc aggaagttcg cggcggcgtc gtcgggccgc      120

ccctcatccc agccctctga cgaggtcctg agcgagttcg agttgcggct gctcagcatg      180

ttcggcctga aacagagacc cacccccagc agggacgccg tggtgccccc ctacatgcta      240

gacctgtatc gcaggcactc aggtcagccg ggctcacccg ccccagacca ccggttggag 300

agggcagcca gccgagccaa cactgtgcgc agcttccacc atgaagaatc tttggaagaa 360

ctaccagaaa cgagtgggaa aacaacccgg agattcttct ttaatttaag ttctatcccc 420

acggaggagt ttatcacctc agcagagctt caggttttcc gagaacagat gcaagatgct 480

ttaggaaaca atagcagttt ccatcaccga attaatattt atgaaatcat aaaacctgca 540

acagccaact cgaaattccc cgtgaccaga cttttggaca ccaggttggt gaatcagaat 600

gcaagcaggt gggaaagttt tgatgtcacc cccgctgtga tgcggtggac tgcacaggga 660

cacgccaacc atggattcgt ggtggaagtg gcccacttgg aggagaaaca aggtgtctcc 720

aagagacatg ttaggataag caggtctttg caccaagatg aacacagctg gtcacagata 780

aggccattgc tagtaacttt tggccatgat ggaaaagggc atcctctcca caaaagagaa 840

aaacgtcaag ccaaacacaa acagcggaaa cgccttaagt ccagctgtaa gagacaccct 900

ttgtacgtgg acttcagtga cgtggggtgg aatgactgga ttgtggctcc cccggggtat 960

cacgcctttt actgccacgg agaatgccct tttcctctgg ctgatcatct gaactccact 1020

aatcatgcca ttgttcagac gttggtcaac tctgttaact ctaagattcc taaggcatgc 1080

tgtgtcccga cagaactcag tgctatctcg atgctgtacc ttgacgagaa tgaaaaggtt 1140

gtattaaaga actatcagga catggttgtg gagggttgtg ggtgtcgcta g 1191

<210> 7
<211> 396
<212> PRT
<213> Homo sapiens

<220>
<221> SOURCE
<222> 1..396
<223> /mol_type="protein"
/note="Sequence of pre-pro-protein of human BMP2 (NP_001191.1)"
/organism="Homo sapiens"

<400> 7
Met Val Ala Gly Thr Arg Cys Leu Leu Ala Leu Leu Leu Pro Gln Val
1               5                   10                  15
Leu Leu Gly Gly Ala Ala Gly Leu Val Pro Glu Leu Gly Arg Arg Lys
            20                  25                  30
Phe Ala Ala Ala Ser Ser Gly Arg Pro Ser Ser Gln Pro Ser Asp Glu
        35                  40                  45
Val Leu Ser Glu Phe Glu Leu Arg Leu Leu Ser Met Phe Gly Leu Lys
    50                  55                  60
Gln Arg Pro Thr Pro Ser Arg Asp Ala Val Val Pro Pro Tyr Met Leu
65                  70                  75                  80
Asp Leu Tyr Arg Arg His Ser Gly Gln Pro Gly Ser Pro Ala Pro Asp
                85                  90                  95
His Arg Leu Glu Arg Ala Ala Ser Arg Ala Asn Thr Val Arg Ser Phe
            100                 105                 110
His His Glu Glu Ser Leu Glu Glu Leu Pro Glu Thr Ser Gly Lys Thr
        115                 120                 125
Thr Arg Arg Phe Phe Phe Asn Leu Ser Ser Ile Pro Thr Glu Glu Phe

130                 135                 140
Ile Thr Ser Ala Glu Leu Gln Val Phe Arg Glu Gln Met Gln Asp Ala
145                 150                 155                 160
Leu Gly Asn Asn Ser Ser Phe His His Arg Ile Asn Ile Tyr Glu Ile
                165                 170                 175
Ile Lys Pro Ala Thr Ala Asn Ser Lys Phe Pro Val Thr Arg Leu Leu
            180                 185                 190
Asp Thr Arg Leu Val Asn Gln Asn Ala Ser Arg Trp Glu Ser Phe Asp
        195                 200                 205
Val Thr Pro Ala Val Met Arg Trp Thr Ala Gln Gly His Ala Asn His
    210                 215                 220
Gly Phe Val Val Glu Val Ala His Leu Glu Glu Lys Gln Gly Val Ser
225                 230                 235                 240
Lys Arg His Val Arg Ile Ser Arg Ser Leu His Gln Asp Glu His Ser
                245                 250                 255
Trp Ser Gln Ile Arg Pro Leu Leu Val Thr Phe Gly His Asp Gly Lys
            260                 265                 270
Gly His Pro Leu His Lys Arg Glu Lys Arg Gln Ala Lys His Lys Gln
        275                 280                 285
Arg Lys Arg Leu Lys Ser Ser Cys Lys Arg His Pro Leu Tyr Val Asp
    290                 295                 300
Phe Ser Asp Val Gly Trp Asn Asp Trp Ile Val Ala Pro Pro Gly Tyr
305                 310                 315                 320
His Ala Phe Tyr Cys His Gly Glu Cys Pro Phe Pro Leu Ala Asp His
                325                 330                 335
Leu Asn Ser Thr Asn His Ala Ile Val Gln Thr Leu Val Asn Ser Val
            340                 345                 350
Asn Ser Lys Ile Pro Lys Ala Cys Cys Val Pro Thr Glu Leu Ser Ala
        355                 360                 365
Ile Ser Met Leu Tyr Leu Asp Glu Asn Glu Lys Val Val Leu Lys Asn
    370                 375                 380
Tyr Gln Asp Met Val Val Glu Gly Cys Gly Cys Arg
385                 390                 395

## Description

Instant invention refers to peptides having the biological activity of a BMP molecule comprising or consisting of an amino acid sequence having an amino acid identity of at least 50% of SEQ ID No. 1, wherein a first amino acid substitution is at position N59 of SEQ ID No. 1 and a second amino acid substitution is at position N102 of SEQ ID No. 1, nucleic acids encoding for the said peptides, vectors comprising the said nucleic acid, transgenic host cells or organisms, a process for the manufacturing of the peptide of the invention, pharmaceutical compositions, research tools or diagnostic tools comprising the molecules, vectors or cells of the invention, the use of the molecules of the invention for the manufacturing of a research tool or diagnostic tool, or for the prevention, treatment or diagnosis of a BMP-related disease or condition.

Bone Morphogenetic Proteins (BMPs) and related Growth & Differentiation Factors (GDFs) are phylogenetically conserved signaling proteins that belong to the Transforming Growth Factor (TGF) beta superfamily. Originally identified for their ability to induce bone formation, they were subsequently shown to be involved in multiple aspects of body patterning and morphogenesis (1). Until now there are more than 20 different BMPs known that have distinct spatiotemporal expression profiles and different functions during embryonic development and in tissue homeostasis. Despite their different functions, all BMPs share a common signaling mechanism. They are translated as precursor proteins consisting of a prodomain, which are released proteolytically after specific cleavage by members of the Subtilisin-like Proprotein Convertase family. The highly conserved mature domain is characterized by seven cysteine (Cys) residues, of which six form an intracellular Cys knot whereas the fourth of the seven Cys is important for the dimerization of BMPs. BMPs are secreted peptides that act as homo- or heterodimers and bind to two major types of membrane-spanning serine/threonine kinase receptors, the type I and type II receptors. Binding of BMPs to preformed heteromeric receptor complexes results in activation of the SMAD and other intracellular pathways.

BMP signaling is precisely regulated by a large number of antagonists, which act extracellularly, on the membrane level and/or intracellularly. A growing number of extracellular antagonists have been identified that bind to BMPs and therefore prevent receptor activation. These include Noggin (NOG), Chordin (CHRD), CHRD-like proteins (CHRDL) and the DAN family including Differential screening-selected gene Aberrant in Neuroblastoma (DAN), Cerberus 1 (CER1), COCO, Protein Related to DAN and CER (PRDC), Gremlin (GREM), Uterine Sensitization-Associated Gene 1 (USAG1), Sclerostin (SOST), Follistatin (FST), FST-like proteins (FSTL), Growth & differentiation factor-Associated Serum Protein 1 (GASP1) and Twisted Gastrulation (TWSG) (2). The inhibitors show distinct expression patterns and are thus likely to have different biological roles in vivo. In addition, antagonists have different affinities for various BMPs and other factors. For example, NOG blocks efficiently BMP2, BMP4, BMP7, GDF5, and GDF6, but not BMP6, BMP9 and BMP10. On the other hand, FST was originally identified as an antagonist of Activins (ACVs), but it has also been shown to bind BMPs. Various BMP antagonists might interact with similar domains in the BMP molecules, given that NOG competes with CER, GREM1 and DAN for binding to BMP2 (3). It was also shown that SOST has pleiotrophic effects, by blocking BMP activity as a BMP antagonist on one hand, but also binding to and neutralizing the activity of NOG, which results in the activation of BMP signaling on the other hand (4).

Thus, the large number of different BMPs is paralleled by about the same number of extracellular antagonists, but detailed knowledge of their biological importance is not available. Until now, there are at least two 3D-structures available of BMPs in complex with an antagonist (BMP7-NOG (PDB: 1 M4U), and Bmp-2 with The First von Willebrand Domain Type C of Crossveinless-2 (POB: 3BK3)). These structures give important information on physical interaction sites, but do not reveal which amino acids are biologically crucial for the interaction. Only few antagonists such as NOG, GREM1, FST, and SOST, have been investigated for their function in skeletal development and regeneration. NOG has a central role in endochondrial ossification as demonstrated by Nog null mice. These mice show a massive expansion of all cartilaginous anlagen as well as a number of other neural tube and brain defects. The importance of NOG for the development of joints was shown by the identification of mutations in NOG in patients with symphalangism and multiple synostosis (multiple joint fusions) syndrome. SOST is mutated in patients with sclerosteosis and van Buchem disease, two conditions characterized by increased bone formation (5).

Since their discovery it has been the intention to use the bone inductive properties of BMPs to promote osteogenesis during fracture healing or to regenerate bone after resection. However, the high potency of BMPs to induce bone in vitro and in animal models can only partly be recapitulated in human patients (6). The BMP-induced effects are likely modulated by several factors such as biological availability, stability and/or local interacting factors in the tissue that inhibit BMPs. Due to the aforementioned reasons, high dosages of protein are required during the treatment of subjects in need, which may lead to unwanted side effects.

Many approaches have been performed to provide improved BMP molecules. For example, it is known from patent application WO2010103070A2 that the substitution of at least two amino acids in the C-terminal region of BMP2 leads to a peptide that is essentially resistant to inhibition by NOG while the biological activity of the molecule is basically maintained.

It is one goal of the present invention to overcome or alleviate any of the aforementioned problems by replacing central residues and domains within Bone Morphogenetic Proteins (BMPs) which are of prime importance for the specific interaction with its natural inhibitor, NOG, and to provide improved BMP molecules for the treatment and prophylaxis of BMP-related diseases or conditions.

Surprisingly, it has been found that the peptides of the invention do not only exhibit an increased specific biological activity of BMP, which is essentially maintained in the presence of the natural BMP-antagonist, Noggin. The peptides of the invention exhibit a reduced susceptibility to inhibition by Noggin, and an increased specific biological activity. The peptides of the invention may further exhibit a reduced susceptibility to the inhibition by Chordin, CHRD-like proteins and members of the DAN family, Cerberus 1, COCO, Protein Related to DAN and CER, Gremlin, Uterine Sensitization-Associated Gene 1, Sclerostin, Follistatin, FST-like proteins, Growth & differentiation factor-Associated Serum Protein 1 and Twisted Gastrulation. Furthermore, the peptides of the invention exhibit an improved stability and/or receptor activation and/or an increased biological half life time in an organism, when compared with wild-type BMP (BMP(wt)), particularly in animals or humans. In addition, the peptides of the invention surprisingly exhibit improved storage stability.

Due to the surprisingly improved specific biological activity and stability of the peptides of the invention, highly reduced amounts of the pharmaceutically effective dosages of BMP proteins (about 1/3 to 1/10 of the amount of BMP(wt)) may be required in the treatment or prophylaxis of BMP-related disorders or conditions.

According to a first aspect the present invention provides a peptide having the biological activity of a BMP molecule comprises or consists of an amino acid sequence having an amino acid identity of at least 50%, preferably at least 60%, more preferred at least 70%, even more preferred at least 80%, particularly preferred at least 90%, more particularly preferred at least 93%, even more particularly preferred at least 95%, especially preferred at least 98%, and most preferred at least 99% of SEQ ID No. 1 (i.e. mature human BMP2), characterized in that said amino acid sequence comprises at least two amino acid substitutions, wherein the first amino acid substitution is at the position corresponding to N59 of SEQ ID No. 1, preferably is selected from N59E, N59K, N59T, and N59V, most preferred is N59V, and wherein the second amino acid substitution is at the position corresponding to N102 of SEQ ID No. 1, preferably is selected from N102S, N102V, and N102W, most preferred is N102W. In a most preferred embodiment of the peptide of the invention, the first amino acid substitution is N59V and the second amino acid substitution is N102W.

As used herein, the term "peptide" encompasses any molecule which essentially comprises a linear chain of natural and/or artificial amino acids which are linked via peptide bonds. Thus, the term "peptide" encompasses oligopeptides, polypeptides, protein fragments, and proteins, wherein, optionally, one or more of the amino acids of the peptide may further be subject to modification. As used herein, reference to amino acids is made by using the single letter code known to the skilled person.

The term "peptide" may mean an isolated peptide that is substantially separated and/or purified from other molecules, which are present in the natural or artificial source of the peptide, preferably from other peptides, in particular from other polypeptides. Optionally, it can be substantially free from cellular material with which it is naturally associated, or culture medium when produced by recombinant techniques, or chemical precursors or other chemicals when chemically synthesized.

In one embodiment of the invention, the term "peptide" means a recombinant expressed peptide. Accordingly, the "peptide" according to the invention may have been altered by human intervention, and/or expressed by a modified cell or organism, preferably a recombinant cell or organism.

In another preferred embodiment of the invention the "peptide" has the biological activity of a BMP molecule, wherein the said BMP molecule is selected from the group consisting of BMP2, BMP4, BMP5, BMP6, BMP7, GDF5, and GDF6, preferably of BMP2, even more preferred of animal and/or human origin, and most preferred of human BMP2.

In accordance with instant invention the term "biological activity of a BMP molecule" may encompass one or more of the biological features of the said naturally occurring BMP/GDF molecules and particularly includes the ability to induce bone and/or cartilage formation, except for the natural susceptibility to its respective natural antagonist or inhibitor NOG. In another embodiment of instant invention the term "biological activity of a BMP molecule" preferably means the ability to activate SMAD signaling, except for the susceptibility with respect to the respective natural antagonist or inhibitor NOG.

In another embodiment of the present invention the peptide having the biological activity of a BMP molecule comprises or consists of an amino acid sequence having an amino acid identity of at least 90% compared to human BMP2 of SEQ ID No. 1, preferably the amino acid identity is at least 93%, more preferably at least 95%, even more preferably at least 98%, most preferably at least 99%.

In still another embodiment of the invention, the peptide having the biological activity of a BMP molecule comprises or consists of an amino acid sequence having an amino acid identity of at least 50%, preferably at least 60%, more preferred at least 70%, even more preferred at least 80%, particularly preferred at least 90%, more particularly preferred at least 93%, even more particularly preferred at least 95%, especially preferred at least 98%, and most preferred at least 99% of SEQ ID No. 7 (i.e. human BMP2 pre-pro-protein), characterized in that said amino acid sequence comprises at least two amino acid substitutions, wherein the first amino acid substitution is at the position corresponding to N341 of SEQ ID No. 7, preferably is selected from N341 E, N341 K, N341 T, and N341 V, particularly preferred is N341 V, and wherein the second amino acid substitution is at the position corresponding to N384 of SEQ ID No. 7, preferably is selected from N384S, N384V, and N384W, particularly preferred is N384W. In a most preferred embodiment of the peptide of the invention, the first amino acid substitution is N341 V and the second amino acid substitution is N384W.

According to the present invention the amino acid identity of peptides is calculated over the whole length of the mature protein of SEQ ID No. 1 or the full length pre-pro-protein of SEQ ID No. 7, respectively, by ignoring (excluding) the at least two amino acid substitutions present in the peptide of the invention. The full length human BMP2 (hBMP2(wt)) according to accession No. NP 001 191 (SEQ ID No. 7) consists of 396 amino acids. N-terminal amino acids 1 to 282, comprising the signal peptide (amino acids 1 to 23) and the pro-domain (amino acids 24 to 282), are cleaved during processing of the full length peptide in order to arrive at the fully processed, active hBMP2 (mature hBMP2) having SEQ ID No. 1. Thus, the amino acid sequence of mature hBMP2 consists of amino acids 283 (Q283) to 396 of the full length pre-pro-protein of hBMP2. As used herein, mature hBMP2 refers to the amino acid sequence of SEQ ID No. 1, which consists of the amino acid sequence of fully processed, active hBMP2 with a total length of 114 amino acids. Accordingly, amino acid 1 of SEQ ID No. 1 refers to amino acid No. 283 of the pre-pro-protein of hBMP2 (SEQ ID No. 7) and amino acid No. 114 refers to amino acid No. 396 of the pre-pro-protein of hBMP2.

The skilled person knows that many BMP molecules are expressed as pre-pro-proteins comprising the above mentioned pro-domain as inserts, which are subject to subsequent processing of the pro-protein in order to obtain the mature protein. Therefore, the skilled person is not bound to the sequence information of the mature peptide, but would consider the insertion of one or more pro-domains into the peptide of the invention. This is to be considered when performing sequence comparisons with respect to the peptide of the invention.

Accordingly, identity or homology of amino acid sequences means the identity of the respective sequences over the whole sequence length in each case (the terms "identity" and "homology" are interchangeably used here within). To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino acid or nucleic acid sequence). The percent identity between the two sequences is a function of the number of identical positions shared by the sequences (i.e., % homology = # of identical positions/total # of positions x 100). The determination of percent homology between two sequences can be accomplished using a mathematical algorithm. A preferred, non-limiting example of a mathematical algorithm utilized for the comparison of two sequences is the algorithm of Karlin and Altschul (7), modified as in (8). Such an algorithm is incorporated into the NBLAST and XBLAST programs (9). BLAST nucleotide searches can be performed with the NBLAST program, score=100, wordlength=12. BLAST protein searches can be performed with the XBLAST program, score=50, wordlength=3. To obtain gapped alignments for comparison purposes, Gapped BLAST can be utilized as described (10). When using BLAST and Gapped BLAST programs, the default parameters of the respective programs (e.g., XBLAST and NBLAST) can be used. Alternatively, identity or homology can be determined, e.g., by comparison with the aid of the ClustalW_Bioedit algorithm (11) using default settings in the software package Bioedit (e.g., available via the world wide web: http://www.mbio.ncsu.edu/BioEdit /bioedit.html).

Preferably, the peptide of the invention exhibits one or more of the biological features of BMP, preferably of a BMP selected from the group consisting of BMP2, BMP4, BMP5, BMP6, BMP7, GDF5, and GDF6, more preferred of BMP2, and even more preferred of animal and/or human origin, and most preferred of human BMP2. The peptide of the invention is classified as exhibiting an increased specific biological BMP activity (of the aforementioned BMP), if the peptide exhibits at least 110% of BMP(wt) activity, i.e. activity of processed, active BMP(wt); preferably, the peptide of the invention exhibits at least 150% of BMP(wt), more preferably of hBMP2(wt) activity. BMP activity can be measured according to any method known to the skilled person. As long as the BMP activity of the peptide of the invention and the control (BMP(wt)) are determined in one and the same test, the exact nature of the BMP activity test is less important. Specific biological activity means the biological activity per amount of protein (g or mol). The skilled person is aware of a number of different test methods, which are suitable to evaluate BMP or BMP2 activity. For example, BMP2 activity may be tested using enzymatic reporters like SMAD-specific induction of a luciferase reporter gene or endogenous induction of alkaline phosphatase expression in ATDC5 or C2C12 cells or by means of the chicken micromass culture system test. The chicken micromass culture system is a well known in vitro model for cartilage differentiation (12; 13), wherein primary mesenchymal cells prepared from chicken limb buds differentiate into chondrocytes. Extracellular matrix production is used as a marker for early chondrogenesis and can be quantified using Alcian blue staining after incubation for three to seven days. To test a specific gene in this system, the cells are infected with a replication competent avian sarcoma (RCAS) virus with the gene of interest incorporated. Overexpression of hBMP2 in the chicken micromass culture system is known to induce cell proliferation and chondrogenic matrix production dramatically (14). In contrast, co-expression of NOG and hBMP2(wt) leads to a complete inhibition of the described differentiation effects.

As used herein, the term amino acid substitution refers to the deletion and/or replacement of a specific amino acid of a given position. In a particular preferred way, the term amino acid substitution can be understood to refer solely to replacement of a specific amino acid of a given position. In order to be specific each substitution is described at least by the type of amino acid to be substituted, preferably in one letter code, and by giving the exact position of said amino acid based on SEQ ID No. 1. A particular substitution can further be specified by naming the amino acid(s) used to replace the amino acid to be substituted. In any case, with a particular substitution only the specified amino acid named before the position number is substituted. A substitution may result in deletion of said amino acid from the sequence, in replacement of said amino acid by exactly one other amino acid and/or in replacement of said amino acid by more than one other amino acid, preferably by not more than three other amino acids, more preferably by two other amino acids.

The peptide of the invention may comprise further amino acid substitutions that are present in addition to the at least two amino acid substitutions as defined above. The peptide of the invention may further contain modifications, for instance mutations that alter protein properties. Such properties may include biological BMP features, e.g. SMAD activity, receptor activation, receptor clearance and biological half life time, resistance to inhibitors or antagonists, biological stability, storage stability, or immunogenicity, or which enable or prevent posttranslational modifications such as PEGylation or glycosylation. Peptides of the invention may be subjected to co- or post- translational modifications, including but not limited to synthetic derivatization of one or more side chains or termini, glycosylation, PEGylation, circular permutation, cyclization, fusion to proteins or protein domains, and addition of peptide tags or labels. The peptide of the invention can be prepared according to known methods. Such methods encompass the synthetic de-novo synthesis of such peptides and/or the expression of the peptides of the invention by means of a nucleic acid encoding such a peptide. In a particular preferred way, the peptide of the invention is prepared by expression using a nucleic acid of the invention. The present invention also refers to a nucleic acid comprising or consisting of a nucleic acid sequence encoding for a peptide of the invention.

According to a second aspect, the present invention provides a nucleic acid comprising or consisting of a nucleic acid sequence encoding for the peptide according to the invention. Preferably, the nucleic acid according to instant invention is a DNA, a cDNA, an RNA, a DNA-RNA-hybrid or a functional equivalent thereof as known in the art.

The term "nucleic acid" according to the present invention encompasses DNA, RNA, hybrids, mixtures and/or functional equivalents thereof, particularly cDNA, genomic DNA and/or RNA which may be of biological origin or (completely or partially) synthetic. The term "nucleic acid" according to the invention encompasses single stranded polynucleotide sequences and (completely or partially) double stranded polynucleotide sequences.

The "nucleic acid" of the invention may be produced by any chemical and/or biological means, including genomic preparations, cDNA preparations, in vitro synthesis, PCR, RT-PCR, and/or in vitro or in vivo transcription.

The "nucleic acid" of the invention may be isolated, which means that it is substantially separated from other molecules, which are present in the natural or artificial source of the nucleic acid, in particular from other nucleic acids, e.g., nucleic acids encoding other polypeptides. The "nucleic acid" may be free of the sequences, which may naturally or artificially flank the nucleic acid (i.e. sequences located at the 5' and 3' ends of the nucleic acid) in its naturally occurring replicon or any other environment. The "nucleic acid" of the present invention may be cloned and may have been altered by human intervention, or placed in an environment that does not correspond to its natural locus or location, or is introduced into a vector or cell.

Moreover, the "nucleic acid" according to instant invention may encompass a cDNA molecule, that can be free from cellular material with which it is naturally associated, or culture medium when produced by recombinant techniques, or chemical precursors or other chemicals when chemically synthesized.

The skilled person is well aware of the degeneracy of the genetic code, allowing for a number of different nucleic acid sequences encoding for the same amino acid sequence and has no difficulties in determining whether a given nucleic acid sequence encodes for an isolated peptide of the invention. The skilled person may adapt the codon usage with respect to the selected host organism or cell when manufacturing the peptide of the invention.

Nucleic acids of the invention can be prepared according to methods known in the art. In a preferred way, nucleic acids of the invention can be prepared by total gene synthesis, or by site-directed mutagenesis of a nucleic acid encoding wild type or modified BMP molecules. Methods may include template-directed ligation, recursive PCR, cassette mutagenesis, site-directed mutagenesis or other techniques that are well known in the art and may be suitable (see for example (16), (17), (18), (19)). The nucleic acid of the present invention may comprise further nucleic acid sequences which may add further functions to the nucleic acid of the invention. For example such additional nucleic acid sequences may include nucleic acid sequences that allow for proper expression of a peptide of the invention and may encompass prodomain-sequences, promoter sequences, regulatory sequences, stop signals, replication origins and the like. The skilled person is well aware of such functional nucleic acid sequences and of how to arrange them in order to arrive at a nucleic acid molecule with the desired properties.

The peptides and/or nucleic acids of the present invention may be provided in its isolated form, i.e. purified from their natural environment, preferably in substantially pure and/or homogeneous form and/or free or substantially free of peptides, nucleic acid other than the desired sequence.

According to a third aspect, the present invention provides a vector comprising or consisting of the nucleic acid of the invention, preferably comprises one or more regulatory elements, which ensure transcription and/or translation in a prokaryotic and/or eukaryotic host cell, especially in a eukaryotic host cell.

According to a forth and a fifth aspect, the present invention provides a transgenic cell and a transgenic organism, each respectively comprising the nucleic acid or the vector of the invention. Usually, the transgenic cell and transgenic organism of the invention is prokaryotic or eukaryotic, preferably eukaryotic. The transgenic cell or transgenic organism of the invention will express one or more peptides of the invention. Preferably, the transgenic organism or cell of the invention is transiently or stably transformed or transfected with one or more nucleic acids of the invention or one or more transgenic expression cassettes comprising a nucleic acid of the invention or one or more vectors encoding for a peptide of the invention or to progeny of such transgenic organisms or cells. Furthermore the present invention relates to cells, cell cultures, tissues and/or parts of transgenic organisms of the invention. It is understood that for the purpose of the present invention the term transgenic organism does not only encompass the organism where the nucleic acid of the invention has been transiently or stably introduced, but also refers to the progeny of such organisms irrespective of the generation order, provided that such progeny comprises the nucleic acid of the invention and/or expresses the peptide of the invention. Preferably the transgenic organism or cell is of prokaryotic or eukaryotic origin, preferably the transgenic organism is a eukaryotic cell. Preferred eukaryotic cells are human cells, animal cells (CHO), such as insect cells (SF6), yeasts (Pichia, Saccharomyces), algae or fungi.

The preparation of a transformed organism or of a transformed cell requires introducing the appropriate DNA into the appropriate host organism or cell. A multiplicity of methods is available for this process which is well known to the person skilled in the art referred to as transformation (20). Thus, by way of example, the DNA may be introduced directly by microinjection or by bombardment with DNA-coated microparticles or nanoparticles. The cell may also be permeabilized chemically, for example using polyethylene glycol, so that the DNA can enter the cell via diffusion. The DNA may also be performed via protoplast fusion with other DNA-containing units such as mini-cells, cells, lysosomes or liposomes. Another suitable method for introducing DNA is electroporation in which the cells are reversibly permeabilized by an electric impulse.

According to a sixth aspect, the present invention provides a process for the manufacturing of the peptide of the invention by use of the nucleic acid of the invention, the vector of the invention, and/or the transgenic cell or organism of the invention comprising the steps of a) optionally, stably or transiently transforming a cell with a nucleic acid of the invention; b) optionally, cultivating the cell obtainable according to step a) under suitable conditions; c) expressing the peptide encoded by the said nucleic acid; d) purifying the peptide from the cultivated cells or the culture medium after having expressed the said peptide.

In an alternative embodiment the process for the manufacturing of the peptide of the invention can be performed by using the nucleic acid of the invention in a system for cell free protein synthesis. In yet another embodiment, the process for the manufacturing of the peptide of the invention can be performed by peptide synthesis. Accordingly, the present invention provides in an alternative embodiment a process for the manufacturing of the peptide of the invention, optionally by use of the nucleic acid of the invention or the vector of the invention in a cell-free peptide synthesis system comprising the steps of a) synthesizing the said peptide encoded by the said nucleic acid under suitable conditions and b) purifying the peptide from the synthesis medium after having synthesized the said peptide.

In yet another embodiment of the invention a peptide according to the invention is provided, which is obtainable by the said manufacturing process using a peptide synthesizer or a cell-free peptide synthesis system, or the said manufacturing process using the nucleic acid, the vector, and/or the transgenic cell or organism of the invention, optionally comprising one or more of steps a) to d): a) stably or transiently transforming a cell with the said nucleic acid; b) cultivating the cell obtainable according to step a) under suitable conditions; c) expressing the said peptide encoded by the said nucleic acid or synthesizing the said peptide encoded by the said nucleic acid under suitable conditions; and d) purifying the said peptide from the cultivated cells or the culture medium after having expressed the said peptide or purifying the said peptide from the synthesis medium after having synthesized the said peptide.

According to a seventh aspect, the present invention provides a pharmaceutical composition comprising or consisting of the peptide of the invention, the peptide obtainable from the manufacturing process of the invention, the nucleic acid of the invention, the vector of the invention or the transgenic cell of the invention and at least one pharmaceutically acceptable carrier or excipient. Preferably, the pharmaceutical composition of the invention is of the sustained-release type, most preferred comprising the peptide of the invention or the peptide obtainable from the manufacturing process of the invention.

According to an eighth aspect, the present invention provides the use of the peptide of the invention and/or the nucleic acid of the invention, which may optionally be labeled, for a) the manufacturing of a monoclonal or polyclonal antibody; b) the screening of nucleic acid libraries; c) the hybridization of a probe; d) the manufacturing of a research tool and/or e) the manufacturing of a diagnostic tool.

According to a ninth aspect, the present invention provides a diagnostic tool or a research tool comprising or consisting of the peptide of the invention, the peptide obtainable from the process of the invention, the nucleic acid of the invention, the vector of the invention, or the transgenic cell of the invention.

The present invention is further directed to the use of the peptide of the invention, the nucleic acid of the invention and/or the transgenic cell or organism of the invention as a medicament.

The present invention is also directed to the use of the peptide, the nucleic acid, the transgenic cell or organism, the pharmaceutical composition, and/or the diagnostic tool of the invention in or for the prevention, treatment or diagnosis of a BMP-related disorder, disease or condition, wherein such disorder, disease or condition includes: any kind of wound repair, formation of bone, formation of cartilage, formation of non-mineralized skeletal or connective tissue; metabolic disease, for example treatment of loss and/or increase of bone mass in metabolic bone diseases (US 5,674,844); replacement or repair of bone and/or cartilage at injury sites such as breaks, fractures and/or tears (US 5,733,878), e.g. repair of the spine or vertebrae; periodontal or dental tissue regeneration (US 5,733,878) including dental implants; regeneration or treatment of tissue from liver (US 5,849,686); pancreas, cardiac, renal, uterine, thyroid, mucous membranes, endothelial, epithelial (skin burns or hair growth); progenitor or bone marrow cells, from tissue of the sensory system such as eye (retina, cornea, optic or olfactory nerve); chronic renal disease or failure (US 6,861,404); enhancement of functional recovery of neuronal tissue including central nervous system ischemia or trauma (US 6,407,060), including neurodegenerative disorders like Parkinson's disease (US 6,506,729), Alzheimer's disease, Huntington chorea disease, multiple sclerosis; dendritic growth (US 6,949,505); neural cell adhesion (US 6,800,603); fibrosis; ulcers, pulmonary artery hypertension, induction of angiogenesis; cancer such as colon or skin cancer; and in the preparation and/or performance of organ or tissue transplantation. According to the understanding of instant invention, the term "treating" encompasses to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein the term "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

The peptide, nucleic acid, and/or transgenic cell of the invention may be used in the manufacturing of a medicament, preferably in the manufacturing of a medicament for the prevention or treatment of a BMP-related disease or condition, wherein such disease or condition comprises wound repair, formation of bone, formation of cartilage, formation of non-mineralized skeletal or connective tissue, metabolic disease, replacement or repair of bone and/or cartilage at injury sites such as breaks, fractures and/or tears, periodontal or dental tissue regeneration, regeneration of tissue from liver, pancreas, cardiac, renal, uterine, thyroid, mucous membranes, endothelium, epithelium, sensory system, progenitor cells, bone marrow cells; chronic renal failure; enhancement of functional recovery of neuronal tissue, dendritic growth, neural cell adhesion; fibrosis; ulcer, pulmonary artery hypertension, induction of angiogenesis; cancer; and in organ or tissue transplantation.

When used in human therapy, the peptide or nucleic acid of the invention and/or their pharmaceutically acceptable salts will generally be administered as a formulation in association with one or more pharmaceutically acceptable excipients. The term "excipient" is used herein to describe any ingredient in the pharmaceutical composition other than the peptide or nucleic acid of the invention. The choice of excipient will to a large extent depend on the particular mode of administration.

If the compounds of the invention shall be administered orally, administration may involve swallowing, so that the compound enters the gastrointestinal tract, or buccal or sublingual administration may be employed by which the compound enters the blood stream directly from the mouth. Formulations suitable for oral administration include: solid formulations such as tablets; capsules containing particulates, liquids, or powders; lozenges (including liquid-filled); and chews; multi- and nanoparticulates; gels; solid solutions; liposomes; films, ovules, sprays and liquid formulations. Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet. The compounds of the invention may also be used in fast-dissolving, fast-disintegrating dosage forms such as those described in the state of the art.

Other possible ingredients include anti-oxidants, colourants, flavouring agents, preservatives and taste-masking agents. The final formulation may comprise one or more layers and may be coated or uncoated; it may even be encapsulated. The formulation of tablets is standard in the art.

Consumable oral films for human use are typically pliable water-soluble or water-swellable thin film dosage forms which may be rapidly dissolving or mucoadhesive and typically comprise isolated peptide or nucleic acid, a film-forming polymer, a binder, a solvent, a humectant, a plasticizer, a stabilizer or emulsifier, a viscosity modifying agent and a solvent.

Some components of the formulation may perform more than one function. The peptide or nucleic acid of the invention may be water-soluble or insoluble.

A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes.

Alternatively, the peptide or nucleic acid of the invention may be in the form of multiparticulate beads. The film-forming polymer may be selected from natural polysaccharides, proteins, or synthetic hydrocolloids and is typically present in the range 0.01 to 99,9% (w/w).

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release.

Modified release formulations include delayed-, sustained-, pulsed-, controlled-, targeted and programmed release. Suitable modified release formulations for the purposes of the invention are, e.g., described in US 6,106,864. Details of other suitable release technologies such as high energy dispersions and osmotic and coated particles are to be found in the art.

The molecules of the invention may also be administered directly into the blood stream, into muscle, into the skeleton or into an internal organ, and, optionally, by means of gene therapy, i.e. administration of a mesenchymal stem cell, which has been transformed by the nucleic acid of the invention. Suitable means for parenteral administration include intravenous, intraarterial, intraperitoneal, intrathecal, intraventricular, intraurethral, intrasternal, intracranial, intramuscular, at the fracture site and subcutaneous. Suitable devices for parenteral administration include needle (including microneedle) injectors, needle-free injectors and infusion techniques. Parenteral formulations are typically aqueous solutions which may contain excipients such as salts, carbohydrates and buffering agents (e.g. at a pH of from 1 to 9, preferably at pH 1 to 7), but, for some applications, they may be more suitably formulated as a sterile nonaqueous solution or as a dried form to be used in conjunction with a suitable vehicle such as sterile, pyrogen-free water. The preparation of parenteral formulations under sterile conditions, for example, by lyophilisation, may readily be accomplished using standard pharmaceutical techniques well known to those skilled in the art. The solubility of the peptide or nucleic acid of the invention used in the preparation of parenteral solutions may be increased by the use of appropriate formulation techniques, such as the incorporation of solubility-enhancing agents. Formulations for parenteral administration may be formulated to be immediate and/or modified release. Modified release formulations include delayed-, sustained-, pulsed-, controlled-, targeted and programmed release. Thus a peptide or nucleic acid of the invention may be formulated as a solid, semi-solid, or thixotropic liquid for administration as an implanted depot or carrier providing modified release of the active compound. Examples of such formulations include drug-coated stents and PGLApoly(dl-lactic-coglycolic)acid (PGLA) microspheres.

The compounds of the invention may be administered topically to or under the skin or mucosa, that is, dermally, transdermally, or subcutaneous. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Optionally, carriers may include alcohol, water, mineral oil, liquid petrolatum, white petrolatum, glycerine, polyethylene glycol and propylene glycol. Penetration enhancers may be incorporated. Alternatively, carriers include solid matrices made from soluble macromolecur entities embedding the compounds of the invention. Accordingly, the compounds of the invention may be combined with soluble macromolecular entities, such as cyclodextrin, collagen, heparin, or polyethylene glycol and suitable derivatives thereof and/or combinations thereof, in order to improve their solubility, dissolution rate, taste masking, bioavailability and/or stability for use in any of the aforementioned modes of administration.

Drug-cyclodextrin complexes, for example, are found to be generally useful for most dosage forms and administration routes, both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in Patent Applications WO91/11172, WO94/02518 and WO98/55148. For administration to human patients, the total daily dose of the compounds of the invention is typically in the range of 0.001 mg to 5000 mg depending, of course, on the mode of administration. For example, an intravenous or locally applied dose may only require from 0.001 mg to 40 mg, the total daily dose may be administered in single or divided doses and may, at the physician's discretion, fall outside of the typical range given herein. These dosages are based on an average human subject having a weight of about 70 kg. The physician will readily be able to determine doses for subjects whose weight falls outside this range, such as infants and the elderly.

In a further aspect, the present invention provides a method of treatment of BMP-related diseases or conditions, wherein such diseases or conditions comprise wound repair, formation of bone, formation of cartilage, formation of non-mineralized skeletal or connective tissue, metabolic disease, replacement or repair of bone and/or cartilage at injury sites such as breaks, fractures and/or tears, periodontal or dental tissue regeneration, regeneration of tissue from liver, pancreas, cardiac, renal, uterine, thyroid, mucous membranes, endothelium, epithelium, sensory system, progenitor cells, bone marrow cells; chronic renal failure; enhancement of functional recovery of neuronal tissue, dendritic growth, neural cell adhesion; fibrosis; ulcer, pulmonary artery hypertension, induction of angiogenesis; cancer; and in organ or tissue transplantation, whereby a subject in need of such treatment is administered an effective dose of a peptide, a nucleic acid and/or a transgenic cell of the invention, optionally together with one or more pharmaceutically acceptable excipients. According to another preferred embodiment of the invention the peptide of the invention may be used for the administration of a therapeutically effective amount or dose of the said peptide in combination with a pharmaceutical carrier of sustained release type.

A sustained release formulation of the compounds of the invention will alleviate the problem of a very high drug load which is present in sustained release formulations of BMP molecules having the specific biological activity of the wild-type peptide.

Sustained release formulations are formulations designed to release a pharmaceutically active compound at the desired rate (i.e. controlled release), e.g., to provide an increased residence time, to maintain the desired level of blood plasma or local concentration of the drug. It is known that controlled release of a drug may be obtained, e. g. by implantable matrix devices, implantable pumps, gels or hydrogels, liposomes, micelles, crystals, microspheres, and reservoir devices. Such implantable matrix devices may include suitable materials containing calcium phosphates such as beta-tricalcium phosphate (Ca₃(PO₄)₂), alpha-tricalcium phosphate and hydroxyl apatite or Ca(OH)₂, coral, natural bone mineral, chitin, non-demineralized bone particles, ceramic bone particles, ceramic dentin, bone chips, bioactive glass, and/or apatite-wollastonite-containing glass ceramic.

E.g., Microspheres are small polymeric particles (e.g., nm to pm in size) wherein a drug is encapsulated, which can be injected, e.g. subcutaneously or intramuscularly. The drug can be released by diffusion, degradation, and water-uptake followed by subsequent diffusion, whereby one or more different polymer materials may be used in a given release system.

Examples of suitable polymers for the manufacturing of microspheres may include poly D, L-lactide-co-glycolide (PLGA), polycarboxyphenoxypropane-co-sebaic acid (pCPP: SA), poly- (fatty acid dimmer-co-sebais acid), poly (trimellitylimidol-1-tyrosine-co-sebaic acid-co-1,3-bis (carboxyphenoxy) propane), polyorthoesters, polyanhydrides, polyamides, polyalkyl-cyanoacrylates and polyphosphazenes, polymethacrylic acid, and block- and/or co-polymers of PLG and PEG.

The drug release rate and profile depend on properties and kind of the microsphere, the type of the polymer and the interaction between the microsphere and the drug. Hydrophilic polymers (such as e.g. PGLA) allow water absorption into the body of the microspheres which results in bulk erosion having a bi-or triphasic release profile. More hydrophobic polymers (such as e.g. pCPP:SA erode from their surface, initially providing a more constant release rate. In case of a biodegradable polymer the rate of the polymer hydrolysis may be the most important factor determining the drug release rate. More labile polymers will give rise to faster release rates.

Specific interactions between polymer and drug may also influence drug release. The size of the microsphere, in particular the surface area-to-volume ratio will have an effect on the rate of the drug release. Various methods to produce microspheres are known in the art. Interfacial polymerisation, in general terms, employs a mixture of monomer(s) and an initiator, where the monomer(s) are polymerised in such a way that the growing polymer forms particles. Three common methods are used, namely suspension, emulsion and dispersion polymerisation. Also extrusion methods are known, to generate microspheres.

Further, hydrogels are used to provide sustained release formulations. Hydrogels are three-dimensional polymer networks, composed of hydrophilic polymers that swell, but do not dissolve in water. The hydrogel network attains physical integrity and is made insoluble due to the presence of chemical and/or physical crosslinks. Hydrogels that are capable of responding to the surrounding environment are termed physiologically responsive hydrogels. Some of the stimuli these hydrogels can respond to are changes in temperature, ionic strength and pH. Some of the most common monomers used to form hydrogels with chemical crosslinks are 2-hydroxyethyl methacrylate, ethylene glycol dimethacrylate, N-isopropyl acrylamide, acrylic acid and methacrylic acid.

Examples of natural polymers from which hydrogels or carriers can be prepared from are alginic acid, carrageenan, chitosan, polylysine, fibrin, collagen, and gelatine.

Most of the above mentioned factors influencing the drug release rate and profile of microspheres do also apply for hydrogels. Various methods to produce hydrogels are known in the art.

Another approach to drug delivery is the use of a polymer matrix in situ from an injected aqueous polymer solution and to use the formed hydrogel as a depot for sustained release of the incorporated therapeutic drug, thus avoiding an invasive surgical (re)placement.

Any drug that is dissolved in the liquid precursor solution is then homogeneously dispersed in the polymer matrix and subsequently released over an extended period of time. An example of in situ forming hydrogel polymer is poly(ethylene glycol)poly(DL-lactic acid-co-glycolic acid) block copolymer. General reference on sustained release formulations is made to Handbook of Pharmaceutical Controlled Release (Wise, D. L., ed. Marcel Dekker, New York, 2000); Drug and the Pharmaceutical Sciences vol. 99: Protein Composition and Delivery (MacNally, E. J., ed. Marcel Dekker, New York, 2000); and Varde et al. in Expert Opin. Biol. Ther., 4,35-51, 2004, which are all incorporated herein by reference.

The term "residence time" is used in its normal meaning, i. e., the time in which a compound is still present in the body or target organ. The residence time is conveniently determined from the time in which the said compound still exerts a pharmaceutical activity. To determine if the peptide of the invention has an increased residence time, e.g., a reduced clearance rate compared to the corresponding BMP(wt), the following experiment is carried out. The two compounds in a suitable buffer is injected into a suitable animal, such as e. g. mice, rats or into humans. Blood samples are collected over time and analyzed for the compound levels as described.

To determine if a formulation gives rise to a sustained release, the following experiment is carried out: The two compounds in a suitable buffer and in the formulation to be tested are injected into a suitable animal, such as e.g. mice, rats or into humans. Blood samples are collected over time and analyzed for the compound levels, as described. As discussed above, a sustained release formulation of a modified BMP protein (BMP variant) so as to give rise to a reduced clearance will alleviate problems associated with the similar formulation of the corresponding unmodified protein. In particular, the high clearance of the unmodified protein dictates a high drug load in the formulation and/or the subject to be treated. The high drug load requires that highly concentrated formulations are made, which is not always possible. In the situations where it is not possible to make formulations with sufficiently high drug loads, larger volumes have to be injected or the injections have to take place more frequently. A therapeutic regime involving the administration of compositions of the present invention will therefore lead to higher convenience to the patient and increased compliance.

Furthermore, the high drug load will give rise to burst effects, i.e. a large release of drug immediately after the injection or implantation. It is known that certain adverse effects are related to such bursts, which should be reduced. Pharmaceutical compositions and formulations according to the present invention have a much lower drug load and will therefore give rise to reduced burst affects. An even greater reduction of the bursts and thus of adverse effects can be achieved if the active compound interacts with the formulation matrix. Examples of such interactions include non-covalent interactions, such as ionic, van der Waals, hydrophobic and hydrogen bond interactions. Such interactions can easily be estimated by simple partition measurements.

In another embodiment, the invention relates to a sustained release formulation comprising the peptide of the invention that has been modified so as to provide interaction of the said peptide with the pharmaceutically acceptable carrier. In particular, said interaction is positive, i.e. there is a (preferably non-covalent) binding between the said peptide and the said carrier.

In still another embodiment, the peptide of the invention is conjugated to PEG, and the sustained release formulation comprises a hydrophobic polymer, such as PEG, PGLA, polymethacrylic acid or co-polymers of the monomers constituting the aforementioned polymers, e.g., tri-block co-polymers such as PLGA-PEG-PLGA.

According to the present invention, the peptide, nucleic acid and/or a pharmaceutical composition of the invention is administered preferably at an effective dose. An "effective dose" is the dose of an active ingredient that upon administration to a patient yields a measurable therapeutical effect with regard to the disease of interest. In the present invention an effective dose is the dose of the isolated peptide or nucleic acid that upon administration to a patient yields a therapeutic effect with regard to one or more of the diseases or conditions specified above in patients suffering there from. Preferably the peptide or nucleic acid of the invention is administered at a dose of not more than 5 mg/kg body weight per treatment or administration. In particular the peptide or nucleic acid of the invention can be administered at a dose of 1 ng/kg to 1 g/kg body weight per treatment or administration, preferably of 0,01 µg/kg to 5000 µg/kg body weight per treatment or administration. In order to prevent acute side effects to occur, it is recommended that the peptide or nucleic acid is administered at a maximum cumulative daily dose of not more than 10 mg/kg body weight.

In the following experimental section, the invention will be explained in further detail by way of figures and examples.

All references, including publications, patent applications, and patents, cited herein are hereby incorporated by reference in their entirety and to the same extent as if each reference were individually and specifically indicated to be incorporated by reference and were set forth in its entirety herein (to the extent permitted by law).

The use of any and all examples, or exemplary language (e.g. , "such as") provided herein, is intended merely to illustrate the invention and does not pose any limitation on the scope of the invention unless otherwise outlined. No language in the specification should be construed as indicating any non-claimed element as essential to the practice of the invention.

### Examples

Throughout the experimental section particular amino acids and amino acid positions are referred to with respect to SEQ ID No. 7, which comprises SEQ ID No. 1.

### Preparation of retroviruses expressing BMP molecules

The coding sequence (cds) of wildtype human BMP2 (SEQ ID No. 6; hBMP2(wt), clone HsCD00080188) is available from DNASU (DNASU, PSI:Biology-MR, Center for Personalized Diagnostics, Biodesign Institute, Arizona State University, Tempe, AZ 85287-6401, USA) and can be amplified by PCR and cloned into the shuttle vector pSLAX13 (Plasmid 13918: pSLAX13 (CT#232)) available from Addgene (Addgene Inc., Cambridge, MA 02139, USA) using a 5'-compatible Ncol-overhang and 3' a BamHI site (24). BMP2 variants were produced by subsequent in vitro mutagenesis of the cds of hBMP2(wt) cloned into pSLAX13 vector using the following mutagenesis primer pairs and the QuikChange® Site-Directed Mutagenesis Kit (Agilent Biotechnologies, Santa Clara, CA, USA):
N341 V fwd: ggctgatcatctgaactccactgttcatgccattgttcaga (SEQ ID NO. 2)
N341 V rev: tctgaacaatggcatgaacagtggagttcagatgatcagcc (SEQ ID NO. 3)
N384W fwd: ccttgacgagaatgaaaaggttgtattaaagtggtatcaggacatggttgtgga (SEQ ID NO. 4)
N384W rev: tccacaaccatgtcctgataccactttaatacaaccttttcattctcgtcaagg (SEQ ID NO. 5)

hBMP2(wt) and BMP2 variants were cloned via Clal into the avian specific retroviral vector RCASBP(A) available from the Retroviral Replication Laboratory, HIV Drug Resistance Program, National Cancer Institute, NCI-Frederick, Frederick, MD 21702-1201, USA as previously described (24).

Shortly, the resulting vectors containing the BMP variants can be transformed into chemically competent E. coli Top10 cells and positive clones can be selected via sequencing.

The cds of human NOG in pLX304 (Gateway V5-tagged lentiviral expression vector)) is available from DNASU (loc. cit.) and can be amplified by PCR and cloned into the shuttle vector pSLAX13 using a Ncol-compatible 5' overhang and a 3'BamHI overhang and subcloned via Clal into the RCASBP(B) available from the Retroviral Replication Laboratory, HIV Drug Resistance Program, National Cancer Institute, NCI-Frederick, Frederick, MD 21702-1201, USA to allow co-infection of BMP2 and NOG in the same cells (24).

For viral production, chicken fibroblast cell line UMNSAH/DF-1 available from ATCC (ATCC® Number: CRL-12203™) can be grown at 37°C to 70% confluence and transfected in a 60mm-cell culture dish with 3 µg RCAS construct and a transfection reagent (Lipofectamine 2000 from Invitrogen) according to manufacturers' instructions. Cells were passaged several times using growth media (DMEM 1 g/I Glucose, w/o L-Gln; 10% FCS; 2% chicken serum; 2 mM L-Gln, Pen/Strep) until 6 cell culture plates of 15 cm diameter showed 100% confluence. Afterwards, the media was changed to starvation media (DMEM 1 g/I Glucose, w/o L-Gln; 1% FCS; 0.2% chicken serum; 2 mM L-Gln, Pen/Strep) leading to an accumulation of viral particles in the media. On 3 consecutive days, the supernatant containing the viral particles was harvested, frozen in liquid nitrogen and stored at -80°C until further use.

The frozen supernatants were thawed at 37°C and filtered on ice through a 0.45 µm Durapore filter (Millipore). Subsequently, the virus particles were pelleted via ultracentrifugation at 22000 rpm (Rotor SW-32, Beckman) for 3 h at 4°C. The supernatant was removed and the pellet resuspended in the remaining media on ice for 1 h by shaking. Finally, the virus was frozen in liquid nitrogen and stored at -80°C.

The virus titer can be determined by seeding UMNSAH/DF-1 cells in a 24 well culture dish plate at a density of 7.6 x 10⁴ cells/well and growing to a confluence of 70-80%. The concentrated viral supernatant was diluted from 1 x 10⁻³ to 1 x 10⁻⁶ and the UMNSAH/DF-1 cells were infected with 1 µl/well and 10 µl/well of the dilution, respectively. Cells infected by the RCAS virus were marked using a monoclonal antibody AMV-3C2 available from the Developmental Studies Hybridoma Bank (University of Iowa, Department of Biology, Iowa City, Iowa, USA) and the Vectastain ABC Kit (Vector Laboratories Inc., Burlingame, CA, USA) and the number of plaque forming units (PFU)/ml of the respective virus was determined.

### In vivo manipulations of chicken limb buds

Injections of RCASBP(A) viruses into HH10 chick embryo hind limb fields was performed as previously described (24, 28). Afterwards embryos were harvested at day 11 of incubation and fixed in 70% ethanol. Skeletal preparation was performed with KOH after staining with Alcian blue and Alizarin red.

### In vitro chondrogenesis assay

Viruses were used to co-infect chicken micromass culture system cultures with hBMP(wt) or hBMP variants with or without NOG. Fertilized chicken eggs (SPF) were obtained from ValoBiomedia, Osterholz-Scharmbeck, Germany) and incubated at 37.5 °C in a humidified egg incubator for 4.5 days. Limb buds of Hamburger/Hamilton stage 24 were isolated and ectoderm was removed by incubation with dispase (3 mg/ml) in HBSS. Cells were isolated from the limb buds by digestion with 0.1% collagenase type la and 0.1 % trypsin followed by filtration of the cell suspension through a 40 µm filter (Becton Dickinson, Falcon^{™}). Micromass cultures were plated at a density of 2x10⁵ cells per 10 µl drop in the centre of a 24-well tissue culture plate. Infection directly prior to plating was performed by adding concentrated replication-competent avian sarcoma (RCAS) viral supernatants: RCASBP(A) containing the cds of hBMP2(wt) or hBMP2 variants and RCASBP(B) containing the cds of wild-type NOG. The cells were allowed to attach for 2 hours in a humidified atmosphere of 5% (v/v) CO₂ at 37°C and then complemented with media (DMEM-F12, 10% FBS, 0.2% chicken serum, 4 mM L-Gln, Pen/Strep). The medium was replaced every 2 days. After 5 days chicken micromass culture system cultures were stained by incorporation of Alcian blue into the extracellular matrix production reflecting proteoglycan-rich cartilaginous matrix after fixation with Kahles Fixative (28.9% (v/v) ethanol, 0.37% (v/v) formaldehyde, 3.9% (v/v) acetic acid) and staining with 0.05% (w/v) Alcian blue in 1 N HCl. Quantification of the staining was achieved by extraction with 6 M guanidine hydrochloride overnight at 22°C. Dye concentration was determined spectrophotometrically at A595nm. For each experiment 3-4 replicates were performed in parallel (13).

### Quantification of human BMP2 protein

A hBMP2 ELISA kit (Antigenix America, Huntington Station, NY 11746, USA) was used for determining BMP2 concentrations in culture supernatants. Nunc Maxisorb^{™} plates were coated overnight at room temperature with a BMP2 capture antibody. All wells were then washed and blocked with coating stabilizer. hBMP2(wt) was serially diluted and incubated on the plate together with the samples to be tested. Following washing of the plate, biotin labeled tracer antibody was added. After application of streptavidin-HRP conjugate and subsequent incubation with TMB substrate, enzyme activity was stopped with sulfuric acid and monitored at A450nm.

### Luciferase reporter assay

HBMP2(wt), hBMP2 variant, NOG and the coding sequences of seven murine TGFβ superfamily type I receptors (Acvrl1, Acvr1, Bmpr1 a, Actr1 b, Tβr1, Bmpr1 b, Acvr1 c) and three type II receptors (Acrv2a, Acvr2b,Bmpr2) can be cloned via Clal into the expression vector pCS2+ for transient transfection of mouse cell lines.

COS-1 cells available from ATCC (ATCC® number: CRL-1650™) or NIH/3T3 cells available from ATCC (ATCC® number: CRL-1658™) were cultured in DMEM glucose 4,5 g/L (Biochrom AG, Berlin, Germany) with 10% FCS and 2mM Gln. 24 h after seeding the cells at a density of 1x10⁴ cells/96-well, the cells were transfected with the indicated constructs. Additionally, co-transfection was performed with a SMAD Binding Element luciferase construct (27) and normalization vector pRL-Tk (Promega Corporation, Madison, WI, USA) using Lipofectamin^{™} 2000 (Invitrogen Corporation, Carlsbad, CA, USA). 42 h later cells were washed once with PBS and lysed in 30 µl potassium phosphate buffer. Luciferase activity was determined as described (30).

### Results

The effect of hBMP2(N341 V-N384W) was evaluated in an in vivo system. Avian specific retroviruses containing the genetic information for hBMP2(wt) and hBMP2(N341 V-N384W) were injected into the right limb field of developing chicken embryos to study the effect of the proteins on limb formation in comparison to the left control extremity. Evaluation of the obtained phenotypes necessitated a distinction between mild, intermediate and strong enhancement of chondrogenesis. A wt-like appearance of the limb was classified as "no enhancement of chondrogenesis", while a paddle-like fusion of all skeletal elements was ranked as "strong". The overall pattern of phenotypes between wild-type and variant was comparable at virus concentrations of 2x10⁷ PFU/ml, indicating a saturation of the system. However, the amount of chicken embryos with severe phenotypes was slightly increased when injecting hBMP2(N341 V-N384W) in comparison to hBMP2(wt). When the titer was decreased to a level of 5x10⁶ PFU/ml, hBMP2(wt) was barely able to enhance chondrogenesis in the developing limb, while the induction of chondrogenesis remained unaltered when injecting the variant. Further reduction of the viral titer to 1x10⁶ PFU/ml resulted in a nearly complete loss of hBMP2(wt) activity, while injection of hBMP2(N341 V-N384W) still caused an enhancement of chondrogenesis in 40% of the embryos. In injections using a high viral titer of 1x10⁸ PFU/ml it could be shown that hBMP2(wt) and hBMP2(N341 V-N384W) showed survival rates of about 70%.

**Tab. 1: Phenotypes resulting from in vivo injection of hBMP2(wt) and hBMP2(N341 V-N384W)**

| Virus titer [PFU/ml] | hBMP2(wt) [%] chondrogenesis trigger effect | | | | hBMP2(N341 V-N384W) [%] chondrogenesis trigger effect | | | |
|---|---|---|---|---|---|---|---|---|
| | strong | medium | mild | none | strong | medium | mild | none |
| 1*10⁶ | 0.00 | 0.00 | 6.25 | 93.75 | 11.54 | 19.23 | 7.69 | 61.54 |
| 5*10⁶ | 0.00 | 0.00 | 15.79 | 84.21 | 28.57 | 34.29 | 31.43 | 5.71 |
| 2*10⁷ | 28.13 | 15.63 | 25.00 | 31.25 | 44.19 | 23.26 | 25.58 | 6.98 |

Both mutations in hBMP2(N341 V-N384W) are located in receptor binding epitopes. N341 is part of the type I receptor binding pocket, while N384 is predicted to interact with the type II receptors. Therefore, we further characterized hBMP2(N341 V-N384W) in comparison to hBMP2(wt) for possible changes receptor specificity using a SMAD-specific luciferase assay in COS-1 cells. In this assay, both hBMP2(wt) and hBMP2(N341 V-N384W) showed comparable activation of the SBE reporter when transfected in a dilution of 1:500 alone or with the four BMP type I receptors and three TGFβ-specific receptors respectively in 10-fold excess. Both BMPs showed a strongly increased signaling when Bmpr1 a or Bmpr1 b were co-transfected. These results indicate that no significant shift in BMP receptor specificity is associated with the mutations N341 V and N384W in hBMP2.

**Tab. 2: Downstream signaling activity of hBMP2(wt) and hBMP2(N341 V-N384W) in COS-1 cells via co-transfected type I receptors**

| Type I receptors | control | hBMP2(wt) | hBMP2(N341 V-N384W) | p-value (T-Test) (wt vs variant) |
|---|---|---|---|---|
| | [rel. luciferase activity (standard deviation)] | | | |
| empty vector | 1.01 (0.14) | 2.51 (0.69) | 2.65 (0.89) | 0,53 |
| Acvrl 1 | 5.65 (1.38) | 6.41 (0.79) | 7.57 (2.22) | 0.47 |
| Acvr1 | 1.15 (0.20) | 1.63 (0.20) | 1.23 (0.18) | 0.06 |
| Bmpr1a | 1.04 (0.33) | 29.62 (6.46) | 24.60 (3.60) | 0.32 |
| ActR1 b | 1.40 (0.06) | 2.81 (0.57) | 3.40 (0.87) | 0.39 |
| TβR1 | 0.94 (0.06) | 2.44 (0.29) | 2.52 (0.31) | 0.76 |
| Bmpr1b | 4.58 (0.33) | 37.49 (3.12) | 28.78 (9.51) | 0.25 |
| Acvr1 c | 1.02 (0.15) | 2.06 (0.38) | 2.75 (0.80) | 0.27 |

Similarly, co-transfection of hBMP2(wt) and hBMP2(N341 V-N384W) with three BMP type II receptors showed no difference in downstream SMAD signaling activation. Both BMPs, used in a dilution of 1:100, strongly induced signaling via the Acvr2 receptor and especially via the Bmpr2 receptor, which were used in a 10fold excess compared to the BMPs.

**Tab. 3: Downstream signaling activity of hBMP2(wt) and hBMP2(N341 V-N384W) in COS 1 cells via co-transfected type II receptors**

| Type II receptors | control | hBMP2(wt) | hBMP2(N341V-N384W) | p-value (T-Test) (wt vs variant) |
|---|---|---|---|---|
| | [rel. luciferase activity (standard deviation)] | | | |
| empty vector | 1.10 (0.13) | 71.12 (12.13) | 64.62 (8.71) | 0.314 |
| Acvr2a | 7.56 (0.93) | 57.35 (5.36) | 46.68 (3.64) | 0.056 |
| Acvr2b | 26.41 (1.32) | 171.28 (5.43) | 156.50 (22.18) | 0.368 |
| Bmpr2 | 11.38 (1.19) | 268.44 (9.98) | 261.56 (26.97) | 0.711 |

As the amino acid exchange N341 V is situated in the NOG interface of hBMP2, we further characterized hBMP2(N341 V-N384W) in comparison to hBMP2(wt) for NOG-insensitivity using a SMAD-specific luciferase assay in NIH3T3 cells. In this assay, hBMP2(wt) and hBMP2(N341 V-N384W) showed comparable activation of the SBE reporter when transfected alone. In contrast to the variant hBMP2(N341 V-N384W), hBMP2(wt) could only induce SBE activation when NOG was not co-transfected and was blocked to control levels in presence of NOG already at very low NOG-encoding DNA concentrations. By contrast, the hBMP2(N341 V-N384W) was blocked to control levels only at a 500-fold surplus of transfected NOG-DNA

**Tab. 4: Downstream SMAD signalling of hBMP2(wt) and hBMP2(N341 V-N384W) in NIH3T3 cells using increasing NOG DNA levels for co-transfection**

| NOG [fold surplus] | control | hBMP2(wt) | hBMP2(N341 V-N384W) | p-value (T-Test) (wt vs variant) |
|---|---|---|---|---|
| | [rel. luciferase activity (standard deviation)] | | | |
| 0x | 1.00 (0.09) | 8.88 (0.13) | 9.81 (0.73) | 0.1548 |
| 5x | 1.46 (0.23) | 1.53 (0.07) | 9.31 (1.14) | 0.0070 (**) |
| 50x | 1.25 (0.09) | 1.51 (0.16) | 7.11 (0.50) | 0.0012 (**) |

The chicken micromass culture system was used to analyse the biological activity of hBMP2(N341 V-N384W) to induce cartilage production in a well defined in vitro system. By incorporation of Alcian blue into the extracellular matrix the production of proteoglycan-rich cartilaginous matrix was determined. The chondrogenic differentiation of the mesenchymal precursor cells in the chicken micromass culture system was visualised and quantified. Hence the potential of hBMP2(N341 V-N384W) to induce chondrogenesis could be efficiently tested. Micromass cultures were retrovirally infected with a virus to express the wild-type protein or its variant hBMP2(N341 V-N384W). Simultaneously, NOG was co-expressed in the cells when the variants were tested for their sensitivity towards the antagonist. After 5 days of cultivation the chicken micromass cultures were stained with Alcian blue. Infection of micromass cells with hBMP2(wt) led to a massive induction of cartilage production compared to the non-infected control cells.

However, hBMP2(wt) was completely blocked when NOG was co-expressed. Like the wild-type, the variant hBMP2(N341 V-N384W) was able to induce chondrogenesis even with a higher efficacy in the absence of NOG. When co-expressed with NOG, the chondrogenic potential was more than 100% of the wild-type activity in the absence of its antagonist.

**Tab. 5: Chondrogenic activity in relation to the amount of BMP2 protein in the absence of NOG**

| Titer 1*10⁷ | chondrog. activity [OD595 nm] (standard deviation) | BMP2 in supernatant [ng] (standard deviation) | chondrog. activity/ ng BMP2 | % |
|---|---|---|---|---|
| hBMP2(wt) | 1.967 (0.01) | 9.78 (1.86) | 0.20 | 100 |
| hBMP2(N341 V-N384W) | 1.977 (0.04) | 2.66 (1.08) | 0.74 | 370 |
| | | | | |
| Titer 1 *10⁶ | | | | |
| hBMP2(wt) | 1.86 (0.04) | 8.09 (1.77) | 0.23 | 100 |
| hBMP2(N341 V-N384W) | 1.74 (0.05) | 2.16 (0.88) | 0.80 | 350 |

In summary, the results show that the combination of the two identified point mutations according to the invention increased NOG resistance of BMP molecules significantly, thereby leading to an improved chondrogenic effect, even more in the presence of its main natural antagonist, NOG. The performed assays confirmed a profound NOG insensitivity and an improved specific biological activity of the BMP variant while the receptor activation profile of the BMP variant was not affected with respect to its main receptors, Bmpr1 a and Bmpr1 b.

References:
(1) Kishigami S, Mishina Y (2005) Cytokine Growth Factor Rev 16: 265- 278
(2) Yanagita M (2005) Cytokine Growth Factor Rev 16: 309-317
(3) Canalis E, Economides AN, Gazzerro E (2003) Endocr Rev 24: 218- 235
(4) Winkler DG et al. (2004) J Biol Chem 279(35):36293-8
(5) Kornak U, Mundlos S. (2003) Sep;73(3):447-74
(6) Groeneveld, EH and Burger EH (2000) Eur J Endocrinol 142:9-21
(7) Karlin and Altschul (1990) Proc. Natl. Acad. ScL USA 87:2264-68
(8) Karlin and Altschul (1993) Proc. Natl. Acad. ScL USA 90:5873-77
(9) Altschul, et al. (1990) J. Mol. Biol. 215:403-10
(10) Altschul et al. (1997) Nucleic Acids Research 25(17):3389-3402
(11) Thompson JO et al. (1994) Nucleic Acids Res 22:4673-4680
(12) DeLise AM et al. (2000) Methods Mol Biol. 137:359-75
(13) Seemann P et al. (2005) J Clin Invest. 2005 Sep;115(9):2373-81
(14) Duprez DM et al. (1996) Dev Biol. Mar 15;174(2):448-52
(15) Sambrook J et al., in Molecular Cloning - A Laboratory Manual, 2nd edition, Cold Spring Harbor Laboratory Press (1989) pages 9.31-9.57 or in Current Protocols in Molecular Biology, John Wiley & Sons, N.Y. (1989), 6.3.1- 6.3.6
(16) Strizhov et al. (1996) Proc Natl Acad Sci USA 93:15012-15017
(17) Prodromou and Perl (1992) Prot. Eng. 5: 827-829
(18) Jayaraman and Puccini (1992) Biotechniques 12: 392-398
(19) Chalmers et al. (2001) Biotechniques 30: 249-252
(20) Keown et al. (1990) Methods in Enzymology 185:527-537
(21) Groppe J et al. (2002) Nature 12;420(6916):636-42)
(22) Allendorph GP et al. (2006) Proc Natl Acad Sci USA 16;103(20):7643-8)
(23) Gao Y, Lai L (2004) J Mol Model 10:44-54)
(24) Morgan BA, Fekete DM. (1996) Methods Cell Biol. 1996;51 :185-218
(25) Guerois R, Nielsen JE, Serrano L (2002) J Mol Biol 320:369-387
(26) Ploeger F et al. (2008) Human Molecular Genetics 17(9):1222-1233
(27) Jonk LJC et al. (1998) J Biol Chem 273(33):21145-21152
(28) Stricker S et al. (2002) Developmental Biology 245(1):95-108
(29) Zimmer J et al. (2012) PloS one, 7(4):e35062
(30) Hampf M, Gossen M., Anal Biochem. (2006) 356(1):94-99

## Claims

1. A peptide having the biological activity of a BMP molecule comprising or consisting of an amino acid sequence having an amino acid identity of at least 50% of SEQ ID No. 1 **characterized in that** said amino acid sequence comprises at least two amino acid substitutions, wherein the first amino acid substitution is at the position corresponding to N59 of SEQ ID No. 1, and wherein the second amino acid substitution is at the position corresponding to N102 of SEQ ID No. 1.

2. The peptide according to claim 1, wherein the first amino acid substitution is selected from N59E, N59K, N59T, and N59V of SEQ ID No. 1 and the second amino acid substitution is selected from N102S, N102V, and N102W of SEQ ID No. 1.

3. The peptide according to claim 1 or 2, wherein the first amino acid substitution is N59V and/or the second amino acid substitution is N102W in SEQ ID No. 1.

4. The peptide according to one or more of claims 1 to 3 having the biological activity of a BMP molecule, wherein the said BMP molecule is selected from the group consisting of BMP2, BMP4, BMP5, BMP6, BMP7, GDF5, and GDF6.

5. The peptide according to claim 4 wherein the said BMP molecule is BMP2.

6. A nucleic acid comprising or consisting of a nucleic acid sequence encoding for the peptide as defined in one or more of claims 1 to 5.

7. A vector comprising or consisting of the nucleic acid as defined in claim 6.

8. The vector according to claim 7 comprising one or more regulatory elements, which ensure transcription and/or translation in a prokaryotic and/or eukaryotic host cell.

9. A transgenic cell or transgenic organism comprising the nucleic acid as defined in claim 6 or the vector as defined in one or more of claims 7 to 8.

10. The transgenic cell or organism according to claim 9 which is prokaryotic or eukaryotic.

11. A process for the manufacturing of the peptide as defined in one or more of claims 1 to 5, by using the nucleic acid as defined in claim 6, the vector as defined in one or more of claims 7 to 8, and/or the transgenic cell or organism as defined in one or more of claims 9 to 10 comprising the steps of
a) optionally, stably or transiently transforming a cell with the said nucleic acid;
b) optionally, cultivating the cell obtainable according to step a) under suitable conditions;
c) expressing the peptide encoded by the said nucleic acid; and
d) purifying the peptide from the cultivated cells or the culture medium after having expressed the said peptide.

12. A pharmaceutical composition comprising or consisting of the peptide as defined in one or more of claims 1 to 5, the peptide obtainable from the process as defined in claim 11, the nucleic acid as defined in claim 6, the vector as defined in one or more of claims 7 to 8 or the transgenic cell as defined in one or more of claims 9 to 10 and at least one pharmaceutically acceptable carrier or excipient.

13. Use of the peptide as defined in one or more of claims 1 to 5 and/or the nucleic acid as defined in claim 6, which may optionally be labeled, for
a) the manufacturing of a monoclonal or polyclonal antibody;
b) the screening of nucleic acid libraries;
c) the hybridization of a probe;
d) the manufacturing of a research tool and/or
e) the manufacturing of a diagnostic tool.

14. A diagnostic tool or a research tool comprising or consisting of the peptide as defined in one or more of claims 1 to 5, the peptide obtainable from the process as defined in claim 11, the nucleic acid as defined in claim 6, the vector as defined in one or more of claims 7 to 8, or the transgenic cell as defined in one or more of claims 9 to 10.

15. Use of the peptide as defined in one or more of claims 1 to 5, the nucleic acid as defined in claim 6, the transgenic cell or organism as defined in claim 9, the pharmaceutical composition as defined in claim 12, and/or the diagnostic tool as defined in claim 14 in or for the prevention, treatment or diagnosis of a BMP-related disease or condition, wherein such disease or condition is selected from wound repair, formation of bone, formation of cartilage, formation of non-mineralized skeletal or connective tissue, metabolic disease, replacement or repair of bone and/or cartilage at injury sites such as breaks, fractures and/or tears, periodontal or dental tissue regeneration, regeneration of tissue from liver, pancreas, cardiac, renal, uterine, thyroid, mucous membranes, endothelium, epithelium, sensory system, progenitor cells, bone marrow cells; chronic renal failure; enhancement of functional recovery of neuronal tissue, dendritic growth, neural cell adhesion; fibrosis; ulcer, pulmonary artery hypertension, induction of angiogenesis; cancer; and in organ or tissue transplantation.
